# EUROPÄISCHE PATENTSCHRIFT

(11) **EP 0 510 480 B1**
(45) Veröffentlichungstag und Bekanntmachung des Hinweises auf die Patenterteilung: **14.12.1994**
(21) Anmeldenummer: 92106352.5
(22) Anmeldetag: 13.04.1992
(51) Int. Cl.: C07C 315/04, C07C 317/36

(54) **Verfahren zum kontinuierlichen Herstellen von Aminoaryl-oder Aminoalkyl-beta-sulfatoethylsulfonen**
Process for the continuous preparation of aminoaryl or aminoalkyl beta-sulfatoethyl sulfones
Procédé pour la préparation en continue d'aminoaryl ou aminoalkyl-beta-sulfatoéthylsulfones

(30) Priorität: 23.04.1991 DE 4113147
(43) Veröffentlichungstag der Anmeldung: 28.10.1992
(73) Patentinhaber: HOECHST AKTIENGESELLSCHAFT, 65926 Frankfurt am Main (DE)
(72) Erfinder: Schmitt, Lothar, W-6240 Königstein/Ts (DE); Berthold, Rüdiger, Dr., W-6232 Bad Soden/Ts. (DE)

(56) Entgegenhaltungen:
- EP-A- 0 144 701
- WO-A-89/05290

## Beschreibung

Die Erfindung betrifft ein Verfahren zum kontinuierlichen Herstellen von Aminoaryl- oder Aminoalkyl-β-sulfatoethylsulfonen durch Umsetzen der entsprechenden β-Hydroxyethylsulfone mit Schwefelsaure im Molverhältnis von 1:1 bis 1:1,15 bei Temperaturen oberhalb 120°C.

Die Herstellung von Aminoaryl- bzw. von Aminoalkyl-β-sulfatoethylsulfone der Formel

NH₂ - A - SO₂ - CH₂ - CH₂ - O - SO₃H

durch Veresterung der entsprechenden β-Hydroxyethylsulfone der Formel

NH₂ - A - SO₂ - CH₂ - CH₂ - OH,

wobei A für einen gegebenenfalls substituierten Benzol- oder Naphthalinrest bzw. für eine Alkylgruppe steht,
mit Schwefelsäure ist bekannt (siehe z.B. WO-A-8 905 290). Die Veresterung erfolgt üblicherweise in beheizten Knetern, auf Trockenbändern oder in Trockenpfannen. Wegen der Temperaturempfindlichkeit bestimmter Produkte darf die Heizmitteltemperatur nur wenig über der Reaktionstemperatur liegen, was zu mehrstündigen Verweilzeiten und schlechter Raum-Zeit-Ausbeute führt. Die Produkte fallen in Form erstarrter Platten (Trockenbänder) oder Granulat mit Knollen bis 40 mm Durchmesser (Kneter, Trockenpfanne) an. Die zementartige Krustenbildung in den Reaktionsapparaten verursacht schlechte Wärmeübergangsbedingungen, was die Reaktionszeit zusätzlich verlängert bzw. die Raum-Zeit-Ausbeute weiter verschlechtert. Darüberhinaus sind für die Antriebe hohe Drehmomente erforderlich, die oft nicht ausreichen, die Reibungskräfte zwischen dem Mischorgan und den Krusten zu überwinden.

Es stellt sich daher die Aufgabe, die Krustenbildung zu verhindern und die Raum-Zeit-Ausbeuten zu verbessern. Diese Aufgabe wird durch ein Verfahren der eingangs genannten Art gelöst, bei dem eine Mischung aus einem β-Hydroxyethylsulfon mit Schwefelsaure einem durch Rezyklieren und mechanischem Rühren erzeugten Wirbelbett aus Endprodukt zugeführt und bei 100 bis 200°C, bevorzugt bei 130 - 180°C, zur Reaktion gebracht wird.

Dabei können 10 bis 50 % des Endproduktes in das Wirbelbett zurückgeführt werden. Die Reaktion der Mischung kann bei Drucken zwischen 50 und 100 mbar durchgeführt werden und einer mittleren Verweilzeit von 15 bis 300 Minuten erfolgen.

Die Vorteile des Verfahrens sind im wesentlichen in der besseren Raum-Zeit-Ausbeute, der kürzeren Verweilzeit und der daraus resultierenden geringeren Temperaturbelastung (höhere Produktqualität) zu sehen. Darüber hinaus wird die Bildung plastischer Phasen und Krusten von Endprodukt vermieden. Durch Variation der Rückführmenge an Endprodukt kann der Reaktorbetrieb in einfacher Weise optimiert werden.

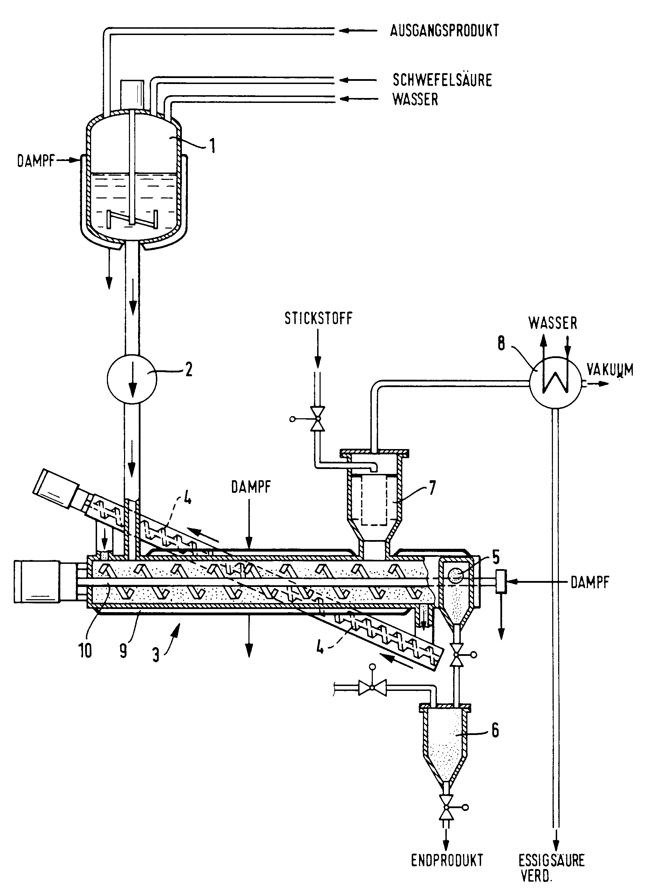

Im folgenden wird das Verfahren anhand der Figur näher erläutert.

Im Rührbehälter 1 werden die Ausgangskomponenten β-Hydroxyethylsulfone und Schwefelsäure im Molverhältnis 1:1 bis 1:1,15 gegebenenfalls unter Zugabe von Wasser gemischt und falls notwendig erwärmt. Mit einer mengeregulierbaren Pumpe 2 wird die Mischung mit konstantem oder pulsierendem Strom in den Reaktor 3 eingespeist. Dort trifft die Mischung auf das vom Rührwerk 10 fluidisierte Granulat, das mit einer Förderschnecke 4 vom Reaktorausgang in den Reaktoreingang zurückgeführt wird. Der Reaktor 3 kann ein System von rotierenden und feststehenden Werkzeugen besitzen, die sich gegenseitig abreinigen und ein ausgeprägtes Zerkleinerungsvermögen aufweisen. Der Mantel 9 des Reaktors und das Rührwerk 10 sind beheizbar und dienen der Erwärmung des Granulats. Die eingepumpte Mischung der Komponenten trifft auf das heiße, vom Rührwerk 10 fluidisierte und von dem Förderorgan 4 recyclierte Granulat. wo es durch direkten Kontakt sehr schnell die Reaktionstemperatur erreicht und erstarrt. Zusammenbackendes Fertigprodukt wird durch die Rührwerkzeuge zu einem Granulat mit hohem Feinanteil zerkleinert.

Die gegebenenfalls in der Ausgangsverbindung enthaltene Acetylgruppe wird während der Reaktion unter Bildung von Essigsäure abgespalten und entweicht gleichzeitig mit dem Wasser. Durch Absenken des Druckes im Reaktionsraum auf 50 bis 150 mbar, vorzugsweise 100 mbar, kann die Reaktion erheblich beschleunigt werden.

Die zweite Hälfte des Reaktors 3 dient als Verweilzeitraum mit der höchsten Temperatur bis 200°C. Die hier zu Ende gehende Reaktion wird durch die Mahlwirkung des Rührwerks 10 weiter aktiviert und gegebenenfalls die Diffusion der Essigsäure und des Wasserdampfes durch Teilchenzerkleinerung und Erzeugung neuer Bruchflächen verbessert. Hier erfolgt also die restlose Entfernung der eingeschlossenen flüchtigen Bestandteile. Die Verweilzeit wird bestimmt durch die in der Höhe verstellbare Austrittsöffnung 5 am Ende des Reaktors 3. Das rieselfähige Endprodukt fließt über die Öffnung 5 in den Zwischenbehälter 6. Die gasförmigen Bestandteile und Inertgase werden über ein Filter 7 geleitet und von mitgerissenem Staub getrennt. Der Staub kann taktweise mittels Inertgas in den Reaktionsraum zurückgeblasen werden. Im Wärmetauscher 8 können die abgetriebenen flüchtigen Bestandteile und Wasser kondensiert und gegebenenfalls danach einer Wiederverwendung zugeführt werden.

### Beispiel:

In einem Reaktor mit selbstreinigendem Rührwerkssystem mit einem Bruttoinhalt von 15,4 l wurde ein Wirbelbett aus Endprodukt durch Vorlegen von granulatförmigem Para-Base-Ester (4-(β-Sulfatoethylsulfonyl)-anilin) hergestellt und unter Rühren auf 140°C geheizt. Danach wurde gleichzeitig granulatförmiges heißes Endprodukt und die auf 80°C vorgeheizte Mischung aus 4-(β-Hydroxyethylsulfonyl)-1-acylaminobenzol, Schwefelsäure und Wasser im Molverhältnis 1:1,05:2,5 in die Einlaufzone des Reaktors eingespeist. Die Zuführrate betrug 6,5 bis 26 kg pro Stunde, die mittlere Verweilzeit des Produkts im Reaktor zwischen 22 und 80 Minuten, der Druck 100 mbar, die Temperatur in der Einlaufzone 125 bis 135°C, in der Mitte des Reaktors 155 bis 165°C, am Austritt 170 bis 180°C und die Rücklaufrate an erzeugtem Produkt 35 %.

Das gewonnene Produkt entsprach der geforderten Qualität. Mit der HPLC-Analyse wurde folgende Zusammensetzung festgestellt:

| | |
|---|---|
| 4-(β-Sulfatoethylsulfonyl)-1-aminobenzol | 97/98 % |
| 4-(β-Hydroxyethylsulfonyl)-1-acetylaminobenzol | < 0,1 % |
| 4-(β-Hydroxyethylsulfonyl)-1-aminobenzol | 0,5/1,3 % |
| 4-(β-Acetoethylsulfonyl)-1-aminobenzol | 0,2/0,4 % |

Unterschiedliche Verweilzeiten, die sich aufgrund der verschiedenen Einspeiseraten ergeben, haben keinen Einfluß auf die Qualitätsdaten. Die physikalischen Reaktionsbedingungen - Temperatur, Druck, Drehzahl - können in einem relativ weiten Bereich ohne signifikanten Einfluß auf die Produkteigenschaften variiert werden.

## Patentansprüche

1. Verfahren zum kontinuierlichen Herstellen von Aminoaryl- oder Aminoalkyl-β-sulfatoethylsulfonen durch Umsetzen der entsprechenden β-Hydroxyethylsulfone mit Schwefelsäure im Molverhältnis von 1:1 bis 1:1,15 bei Temperaturen oberhalb 120°C, dadurch gekennzeichnet, daß eine Mischung aus einem β-Hydroxyethylsulfon und Schwefelsäure einem durch Rezyklieren und mechanischem Rühren erzeugten Wirbelbett aus Endprodukt zugeführt und bei 100 bis 200°C, bevorzugt bei 130 bis 180°C zur Reaktion gebracht wird.

2. Verfahren nach Anspruch 1, dadurch gekennzeichnet, daß 10 bis 50 % des Endproduktes in das Wirbelbett zurückgeführt werden.

3. Verfahren nach Anspruch 1 oder 2, dadurch gekennzeichnet, daß die Umsetzung der Mischung bei Drucken zwischen 50 und 150 mbar erfolgt.

4. Verfahren nach einem der Ansprüche 1 bis 3, dadurch gekennzeichnet, daß die Umsetzung der Mischung bei mittleren Verweilzeiten von 15 bis 300 Minuten erfolgt.

## Claims

1. A process for the continuous preparation of aminoaryl or aminoalkyl β-sulfatoethyl sulfones by reaction of the corresponding β-hydroxyethyl sulfones with sulfuric acid in a molar ratio of 1:1 to 1:1.15 at temperatures above 120°C, wherein a mixture of a β-hydroxyethyl sulfone with sulfuric acid is supplied to a fluidized bed of end product produced by recycling and mechanical stirring and is reacted at 100 to 200°C, preferably at 130 to 180°C.

2. The process as claimed in claim 1, wherein 10 to 50% of the end product is recycled into the fluidized bed.

3. The process as claimed in claim 1 or 2, wherein the reaction of the mixture takes place under pressures of between 50 and 150 mbar.

4. The process as claimed in one of claims 1 to 3, wherein the reaction of the mixture takes place with average residence times of 15 to 300 minutes.

## Revendications

1. Procédé pour préparer en continu des aminoaryl- ou des aminoalkyl-β-sulfatoéthyl sulfones par la réaction des β-hydroxyéthyl sulfones correspondantes avec de l'acide sulfurique selon le rapport molaire de 1:1 à 1:1,15, à des températures supérieures à 120°C, procédé caractérisé en ce qu'on achemine un mélange d'une β-hydroxyéthyl sulfone et d'acide sulfurique à un lit fluidisé du produit final, lit obtenu par recyclage et agitation mécanique, et l'on fait réagir ce mélange à 100 jusqu'à 200°C, avantageusement à 130, jusqu'à 180°C.

2. Procédé selon la revendication 1, caractérisé en ce qu'on recycle dans le lit fluidisé 10 à 50 % du produit final.

3. Procédé selon la revendication 1 ou 2, caractérisé en ce que la réaction du mélange a lieu sous des pressions comprises entre 50 et 150 mbars.

4. Procédé selon l'une des revendications 1 à 3, caractérisé en ce que la réaction du mélange a lieu avec des temps moyens de séjour de 15 à 300 minutes.
